(19) **Europäisches Patentamt**
**European Patent Office**
**Office européen des brevets**

(11) **EP 2 703 004 B1**

(12) **EUROPEAN PATENT SPECIFICATION**

(45) Date of publication and mention
of the grant of the patent:
**06.05.2015 Bulletin 2015/19**

(51) Int Cl.:
***A61K 36/258*** (2006.01)    ***A61P 15/00*** (2006.01)
***A61K 31/198*** (2006.01)

(21) Application number: **13189406.5**

(22) Date of filing: **28.05.2008**

(54) **Composition for improving male sexual function containing ginseng berry extract**

Zusammensetzung zur Verbesserung der männlichen Sexualfunktion mit Ginsengbeerenextrakt

Composition pour la amélioration de la fonction sexuelle masculine contenant de l'extrait de baie de ginseng

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **28.05.2007 KR 20070051593**
**09.05.2008 KR 20080043488**

(43) Date of publication of application:
**05.03.2014 Bulletin 2014/10**

(62) Document number(s) of the earlier application(s) in accordance with Art. 76 EPC:
**08765961.1 / 2 152 281**

(73) Proprietor: **Amorepacific Corporation**
**Seoul 140-777 (KR)**

(72) Inventors:
• **Park, Chan-Woong**
**Gyeonggi-do 446-729 (KR)**
• **Yeom, Myeong Hoon**
**Gyeonggi-do 446-729 (KR)**
• **Cho, Nam Hoon**
**Gyeonggi-do 446-729 (KR)**
• **Lee, Sang Jun**
**Gyeonggi-do 446-729 (KR)**
• **Lee, Sang Min**
**Yongin-si,Gyeonggi-do 446-729 (KR)**
• **Lee, Jin Young**
**Gyeonggi-do 446-729 (KR)**
• **Jeon, Hee Yong**
**Gyeonggi-do 446-729 (KR)**
• **Kim Young-Myeong**
**200-180 CHUNCHEON-SI GANGWON-DO (KR)**

• **Kim, Chun-Ki**
**200-161 CHUNCHEON-SI GANGWON-DO (KR)**
• **Choi, Young Deuk**
**135-110 SEOUL (KR)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
**JP-A- 2000 212 080**

• **RYU J-K ET AL: "Free radical-scavenging activity of Korean red ginseng for erectile dysfunction in non-insulin-dependent diabetes mellitus rats", UROLOGY, BELLE MEAD, NJ, US, vol. 65, no. 3, 1 March 2005 (2005-03-01), pages 611-615, XP004789802, ISSN: 0090-4295, DOI: 10.1016/J.UROLOGY.2004.10.038**
• **CHEN X ET AL: "Ginsenosides -induced nitric oxide-mediated relaxation of the rabbit corpus cavernosum", BRITISH JOURNAL OF PHARMACOLOGY, NATURE PUBLISHING GROUP, BASINGSTOKE, HANTS; GB, vol. 115, no. 1, 1 May 1995 (1995-05-01), pages 15-18, XP002110278, ISSN: 0007-1188**
• **SOO YEON KANG ET AL: "GINSENOSIDES OF THE PROTOPANAXATRIOL GROUP CAUSE ENDOTHELIUM-DEPENDENT RELAXATION IN THE RAT AORTA", LIFE SCIENCES, PERGAMON PRESS, OXFORD, GB, vol. 56, no. 19, 1 January 1995 (1995-01-01), pages 1577-1586, XP001162793, ISSN: 0024-3205, DOI: 10.1016/0024-3205(95)00124-O**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 703 004 B1

- **SHAO Z-H ET AL: "Antioxidant effects of American ginseng berry extract in cardiomyocytes exposed to acute oxidant stress", BIOCHIMICA ET BIOPHYSICA ACTA (BBA) - GENERAL SUBJECTS, ELSEVIER, AMSTERDAM, NL, vol. 1670, no. 3, 24 February 2004 (2004-02-24), pages 165-171, XP004491176, ISSN: 0304-4165, DOI: 10.1016/J.BBAGEN. 2003.12.001**
- **MEHENDALE S R ET AL: "Chronic pretreatment with American ginseng berry and its polyphenolic constituents attenuate oxidant stress in cardiomyocytes", EUROPEAN JOURNAL OF PHARMACOLOGY, ELSEVIER SCIENCE, NL, vol. 553, no. 1-3, 28 December 2006 (2006-12-28), pages 209-214, XP028029383, ISSN: 0014-2999, DOI: 10.1016/J.EJPHAR.2006.09.051 [retrieved on 2006-12-28]**

Remarks:
The file contains technical information submitted after the application was filed and not included in this specification

2

## Description

Technical Field

The present disclosure comprising the invention relates to a composition for facilitation of blood circulation, a composition for prevention of vascular aging, a composition for treatment of vascular inflammation, a composition for facilitation of angiogenesis, a composition for treatment of ischemic heart disease, a composition for improvement and treatment of local blood circulation insufficiency, a composition for improvement of skin beauty, and a composition for improvement of male sexual function, which comprise ginseng berry extract as active ingredient. More particularly, the present disclosure comprising the invention relates to compositions comprising ginseng berry extract as active ingredient, which facilitate generation of nitric oxide (NO) in endothelial cells, improve viability of endothelial cells, increase mobility of endothelial cells and facilitate angiogenesis through vascular tube formation; relax corpus cavernosum and improve penial erection through facilitating generation of NO in endothelial cells, thereby improving male sexual function; and provide antioxidative effect, prevent skin aging and improve wrinkles through facilitation of collagen biosynthesis and prevention of MMP-1, provide skin whitening effect through inhibition of melanin synthesis, and provide skin moisturizing effect.

Background Art

Ginseng (*Panax ginseng* C.A. Meyer) is a plant belonging to the genus ginseng, in the family Araliaceae, and has been used as herb from over 2,000 years in Korea, China, Japan and other countries. Empirically, it has been used to prevent diseases and extend life span. Until now, ginseng is known to have the following effects: positive effect on the central nervous system, anti-carcinogenetic effect, anticancer activity, immune function control, antidiabetic effect, liver function improving effect, improvement of cardiovascular disorder, anti-arteriosclerotic effect, blood pressure control, improvement of climacterium, improvement of osteoporotic conditions, antistress and anti-fatigue effects, antioxidative effect, aging prevention effect, and the like [The Recent Korean Ginseng: Constituents and Effects, Korea Ginseng and Tobacco Research Institute, 56-112, 1996].

Ginsenosides, which are typical active compounds of ginseng, are uniformly distributed in aerial and underground parts of the plant. Particularly, it is known that not only contents but also compositions of ginsenosides are different depending on parts from ginseng root to ginseng leaf to ginseng berry (Attele AS et al, Biochem. Pharmacol., 58; 1685-1693, 1999). Among them, ginseng berry is reported to provide superior antidiabetic effect to ginseng root, with characteristic content and composition of ginsenosides (Dey L. et al., Phytomedicine, 10; 600-605, 2003).

From old times, ginseng berry has been valued more preciously than other parts of ginseng. It has been selected and harvested to obtain seeds. Seed gathering from ginseng berry is carried out only once in 4-year-old ginseng. It is difficult to produce good yearlings from 3-year-old ginseng, because the seeds are too small. Seeds gathered from ginseng 5 or more years old are robust, but the ginseng root may not grow sufficiently, and it is difficult to produce high-quality red ginseng because the tissue is not so dense. And, if seed gathering is carried out 2 or more times, quantity and quality of red ginseng are impaired significantly [The Recent Korean Ginseng: Cultivation, Korea Ginseng and Tobacco Research Institute, 130-131, 1996].

With regard to blood circulation, dilation of capillaries is essential particularly in peripheral blood circulation. That is, increase of blood flow in the blood vessels is impossible without dilation of the blood vessels. With regard to dilation of blood vessels, NO is involved by the action of eNOS (endothelial nitric oxide synthase). Therefore, in case of hypertension, generation of NO decreases [Forete, P. et al., Basal nitric acid synthesis in essential hypertension. Lancet. 1997;349:837-842]. Other factors such as aging, smoking, hyperlipemia and diabetes reduce NO generation in blood vessels [Crossman, DC. More problems with endothelium. Q J Med. 1997; 90:157-160].

Angiogenesis is a process involving the growth of new blood vessels from pre-existing vessels. It occurs in several stages migration of endothelial cells constituting blood vessels, invasion through extracellular matrix (ECM), which is an inter-cellular barrier, proliferation, and differentiation into blood vessels (tube formation) [Folkman, J. et al., Angiogenesis. The Journal of Biological Chemistry, 1992, 267(16), 10931-10934].

Physiologically, angiogenesis occurs during embryonic development or menstruation and may occur temporarily due to local oxygen deficiency. Therapeutic angiogenesis is utilized in case blood flow is insufficient as in ischemic disease, bone fracture, etc. Worldwide, ischemic cardiovascular diseases caused by arteriosclerosis are among the major cause of deaths, and they are increasing fast in Korea, too [Jeong, Jin-Ok et al., Therapeutic angiogenesis. Journal of Korean Society for Vascular Surgery. 2000. 16(2), 265-269).

1-Arginine is a basic amino acid with chemical formula $C_6H_{14}N_4O_2$ and molecule weight 174.21. It was first isolated from a lupin (a kind of bean) seedling extract. 1-Arginine is one of the amino acids constituting proteins. It is rich in the protein protamine, which exists in the sperm of fish, and exists in free state in plant seeds. Further, it is a major component in the urea cycle (also known as the ornithine cycle). By the action of the enzyme arginase, it is decomposed into urea and ornithine. It is synthesized from citrulline and aspartic acid. Although 1-arginine is a nonessential amino acid in adults, it is nutritionally essential in infants.

NO-nitro-1-arginine is known as an inhibitor of nitric oxide synthase. Researches show that NO-nitro-1-arginine may interfere with the relaxation of blood vessels. However, other researches show that the inhibition effect of NO-nitro-1-arginine can be reversed in the presence of 1-arginine ($3 \times 10^{-3}$ mol/L) [Simonsen et al., Nitric oxide is involved in the inhibitory neurotransmission and endothelium-dependent relaxations of human small penial arteries, Clin. Sci. 92:3, 265-75.]. This research asserts that 1-arginine can be an effective substrate for nitric oxide synthase and can stimulate of release of free NO in blood vessels.

The aspects of male sexual function include sexual desire, penial erection, ejaculation and orgasm. This sexual function is determined by complicated physiological interactions of the nervous, endocrine and blood circulatory systems. A disorder in any of them may result in sexual dysfunction. Until just about 10 years ago, sexual dysfunction has been considered to result from psychogenic reasons. However, with the development of modem medical science, it has been found that sexual dysfunction is caused by various reasons including disorders in the blood circulatory, nervous and endocrine systems, diabetes, hypertension, drug intake, and the like in about 50% of patients. Recently, sildenafil, which is an inhibitor of phosphodiesterase V, is drawing a lot of interests with respect to treatment of sexual dysfunction. But, this therapy merely induces erection temporarily using a chemical, and is costly and associated with a lot of adverse reactions, including headache, increased blood pressure, heart attack, and the like. Especially, not a few deaths cases associated with heart attack are reported. Accordingly, a safe and effective treatment that can enhance the erectile function fundamentally is required. The recent trend is toward the development of sexual dysfunction treatment which increases the production of NO and cGMP, which are signal transduction substances that induce strong relaxation of the cavernous smooth muscle, and, thereby, enhances penial erection.

The changes occurring during penial erection are complicated and require a highly coordinated control involving the peripheral and central nervous systems and the endocrine system. The contraction of the cavernous smooth muscle is controlled by noradrenergic nerve stimulation through activation of post-synaptic $\alpha$1 adrenergic receptor, and the erectile dysfunction may be associated with the increased tension of the cavernous smooth muscle. However, relaxation of the penial smooth muscle is mediated in part by the non-adrenergic, non-cholinergic (NANC) neurotransmission, and the decrease of tension of the penial cavernous smooth muscle is caused by the relaxation of the corpus cavernosum by NO. During sexual excitement, NO is released from neurons and endothelial cells, binds with soluble guanylate cyclase (sGC) existing in smooth muscle cells and endothelial cells and activate it, and, thereby, increases the level of cyclic guanosine 3'-,5'-monophosphate (cGMP) in the cells. Through unknown mechanism, although it is believed that activation of protein kinase G is associated, the increased cGMP level induces the relaxation of the corpus cavernosum by reducing calcium level in the cells (it is probable that it is caused by the activation of $Ca^{2+}$-activated $K^{+}$-channel) [Chuang et al., cGMP mediates corpus cavernosum smooth muscle relaxation with altered cross-bridge function. Life Sci. 1998;63(3):185-94].

The present invention is defined by the claims. The inventors of the present invention have found out that the extract of ginseng berry, which is an aerial part of ginseng, has different composition and effect from other parts, including ginseng root or red ginseng root.

Accordingly, an object of the present invention is to provide a composition for improvement of male sexual function through increased generation of NO, which is a signal transduction substance that induces strong relaxation of the cavernous smooth muscle, and, thereby, enhanced penial erection, ginseng berry extract as active ingredient.

Technical Solution

In another aspect, the present invention provides a composition for improvement of male sexual function comprising ginseng berry extract as active ingredient. In another aspect, the present invention provides a composition for improvement of male sexual function comprising ginseng berry extract as active ingredient and further comprising 1-arginine.

Advantageous Effects

The composition comprising ginseng berry extract according to the present invention dilates blood vessels, thereby facilitating blood circulation, inhibits vascular aging, treats vascular inflammation, is useful in treatment of ischemic heart diseases such as arteriosclerosis, angina pectoris and myocardial infarction through facilitation of angiogenesis, and is useful in improvement and treatment of local blood circulation insufficiency caused by arthritic inflammation or fracture. Further, it has superior skin aging inhibition effect, skin wrinkle improving effect, skin whitening effect and skin moisturizing effect. Therefore, health functional food for improving skin beauty can be prepared using the composition according to the present invention. Further, the ginseng berry extract according to the present invention increases generation of NO in endothelial cells and, thus, provides the possibility of improving penial erection. This effect may be further improved when it is used in combination with 1-arginine, which is a substrate of nitric oxide synthase. Accordingly, a composition comprising ginseng berry extract and 1-arginine as active ingredient may effectively improve male sexual function.

Description of Drawings

The above and other objects, features and other advantages of the present invention will be more clearly understood from the following detailed description taken in conjunction with the accompanying claims, in which:

Figure 1a is a confocal laser microscopic image of endothelial cells, and Figure 1b compares intensity of fluorescence with that of the non-treated group;
Figure 2a shows proliferation of surviving endothelial cells, and Figure 2b compares absorbance of stained endothelial cells;
Figure 3a shows mobility of stained endothelial cells, and Figure 3b compares mobility of the cells with that of the non-treated group;
Figure 4a shows fixed, stained endothelial cells, and Figure 4b compares tube length with that of non-treated group;
Figure 5a is an optical microscopic image showing new capillary vessels extending from the aortic ring, and Figure 5b shows vessel sprouting promoting effect evaluated by measuring length and number of newly formed vessels;
Figure 6a is a real-time, intravital fluorescence microscopic image of angiogenesis in a mouse, and Figure 6b shows blood vessel change analyzed using a computer program (0: fewest changes, 5: most changes);
Figure 7 compares inhibition of LPS-induced NO generation;
Figure 8 compares generation of NO and inflammatory factors PGE2, TNF-$\alpha$ and IL1-$\beta$ in mouse blood;
Figure 9 shows reactive oxygen species removal activity of test substances;
Figure 10 shows collagen generation of the ginseng berry extract of Example 1;
Figure 11 shows MMP-1 synthesis inhibition effect of the ginseng berry extract of Example 1;
Figure 12 shows wrinkle inhibition effect of the ginseng berry extract of Example 1;
Figure 13 shows wrinkle decrease effect of the ginseng berry extract of Example 1;
Figure 14 is a confocal laser microscopic image showing NO generation facilitating effect of 1-arginine, red ginseng extract and ginseng berry extract in endothelial cells;
Figure 15 is a graph showing NO generation facilitating effect of 1-arginine, red ginseng extract and ginseng berry extract in endothelial cells as relative fluorescence intensity; and
Figure 16 is a graph showing synergic NO generation facilitating effect of ginseng berry extract and 1-arginine as relative fluorescence.

Best Mode

Hereinafter, reference will be made in more detail to the present invention. The composition of the present invention comprises ginseng berry extract as active ingredient in order to improve penial erection through increased NO generation in endothelial cells and, thereby, improve male sexual function, and may further comprise 1-arginine.

In order to improve male sexual function, it is required to facilitate penial erection through strong relaxation of the corpus cavernosum. It is related with increasing generation of NO. In animal experiments, it was found out that NO plays an important role in penial erection. Upon sexual stimulation, NO generation in endothelial cells increases at the penial parasympathetic periphery. NO activates guanylate cyclase, which converts guanosine triphosphate (GTP) to cyclic guanosine monophosphate (cGMP). The resultant cGMP provides a signal which triggers relaxation of cavernous smooth muscle and penial artery, thereby inducing penial erection. Accordingly, generation of NO is critical to sustain penial erection. Therefore, a combined use of 1-arginine, which is the substrate of nitric oxide synthase, and the material which facilitates generation of NO in blood vessels will trigger sufficient release of NO in the corpus cavernosum, thereby inducing relaxation of smooth muscle, consistent inflow of blood and treatment of erectile dysfunction.

The composition for improvement of male sexual function according to the present invention may comprise 0.01-100 weight% of ginseng berry extract based on the total weight of the composition, depending on the composition type. And, when 1-arginine is used in combination, the 1-arginine may be included in an amount of 0.01-99.9 weight% based on the total weight of the composition.

The composition comprising ginseng berry extract of the present invention may be used as additive for food, medicine, and the like.

When used for medicine, the composition comprising ginseng berry extract as active ingredient according to the present invention may be prepared into solid, semisolid or liquid form for oral or parenteral administration, by adding a commonly used inorganic or organic carrier.

Preparation forms for oral administration may include tablet, pill, granule, soft/hard capsule, powder, fine granule, emulsion, syrup, pellet, and the like. Preparation forms for parenteral administration may include injection, drop, ointment, lotion, spray, suspension, emulsion, suppository, and the like. The active ingredient of the present invention may be prepared into a preparation form by a method commonly used in the art. Surfactant, excipient, colorant, fragrance, preservative, stabilizer, buffer, suspending agent, or other adjuvants may be used adequately.

The composition of the present invention may be prepared into a pharmaceutical formulation according to a commonly used method. When preparing the formulation, it is preferable that the active ingredient is mixed or diluted with a carrier, or encapsulated in a receptacle-type carrier. In case a diluent is used as carrier, it may be a solid, semisolid or liquid substance which acts as carrier, excipient or medium for the active ingredient.

Examples of adequate carrier, excipient or diluent include lactose, dextrose, sucrose, sorbitol, mannitol, calcium silicate, cellulose, methyl cellulose, microcrystalline cellulose, polyvinylpyrrolidone, water, methylhydroxybenzoate, propylhydroxybenzoate, talc, magnesium stearate and mineral oil. The formulation may further include filler, antiflocculant, lubricant, humectant, fragrance, emulsifier, antiseptic, or the like. The composition of the present invention may be formulated by the method well known in the art so that the active ingredient can be released in an immediate, sustained or controlled manner after being administered to a mammal.

The pharmaceutical composition of the present invention may be prepared through various routes, including orally, intradermally, subcutaneously, intravenously, intraperitoneally, intramuscularly, through topical application, using a patch, or iontophoretically. Among them, topical application and oral administration are preferred.

For human, a daily dosage of the active ingredient may be 1 to 100 mg/kg body weight, preferably 5 to 70 mg/kg body weight. Administration may be made once or several times a day. However, the actual dosage of the active ingredient should be determined considering various factors, including particular disease to be treated, administration route, age, sex and body weight of the patient, severity of the disease, and the like. Accordingly, the afore-mentioned administration dosage by no means limits the scope of the present invention.

When used for functional food, the composition comprising ginseng berry extract as active ingredient according to the present invention may be prepared into tablet, capsule, soft capsule, pill, granule, drink, diet bar, chocolate, caramel, confectionery, etc. by adding a component commonly used in the related art. Further, functional ingredients suitable for functional food may be added adequately.

Mode for Invention

The following examples and comparative examples illustrate the present invention in more detail, but are not intended to limit the scope of the present invention.

Example 1: Preparation of ginseng berry extract

1) Pre-treatment of ginseng berry: Seeds were separated and removed from harvested raw ginseng berry. Pulp and pericarp of ginseng berry were dried using sunlight or hot air to obtain dried ginseng berry.
2) Preparation of ginseng berry extract: After adding 3 L of ethanol to 1 kg of dried ginseng berry, 300 g of ginseng berry extract was obtained by extraction under reflux, followed by filtration and concentration under reduced pressure at 40-45 °C.

Example 2: Preparation of ginseng berry extract

1) 100 g of the ginseng berry extract obtained in Example 1 was dissolved in 1 L of water. Oil-soluble constituents were removed using a separatory funnel, by adding 500 mL of diethyl ether. Then, 500 mL of water-saturated butanol was added to the remaining water layer. This procedure was repeated 3 times. The resultant butanol layer was concentrated under reduced pressure to obtain 60 g of ginseng berry extract (crude saponin).

Comparative Example 1: Preparation of ginseng root extract

Ginseng root extract was prepared in a manner similar to Example 1, except for using red ginseng root instead of ginseng berry.

Test Example 1: Comparison of components of ginseng berry extract and ginseng root extract

1. Analysis of ginsenoside (ginseng saponin) component

Each of the ginseng berry extract and the ginseng root extract prepared in Example 1 and Comparative Example 1, respectively, was treated with ether to remove oil-soluble constituents. Then, crude saponin was extracted with butanol (BuOH) and concentrated. Analysis of ginsenoside (ginseng saponin) component was carried out using HPLC. The result is given in the following Table 1.

Table 1

|  | Example 1 | Comparative Example 1 |
|---|---|---|
| Crude saponin content(total ginseng saponin, dry weight) | 33.42% | 16.70% |
| PD/PT | 0.73 | 3.23 |

The ginseng berry extract prepared in Example 1 had about twice crude saponin as compared with the ginseng root extract prepared in Comparative Example 1. Ginsenoside compositions of the ginseng berry extract and the ginseng root extract were distinctly different, as can be seen from the ratio of PD (protopanaxadiols; ginsenosides Rb1, Rb2, Rc and Rd) and PT (protopanaxatriols; ginsenosides Re, Rg1 and Rg2), which was 0.73 and 3.23, respectively.

2. Analysis of mineral component of ginseng berry extract

Assuming that the ginseng berry extract prepared in Example 1 would include vitamin and mineral components that are not or hardly included in ginseng root, component analysis was carried out. The result is given in the following Table 2.

Table 2

| Components | Contents | Components | Contents |
|---|---|---|---|
| Potassium (mg/100 g) | 5865.57 | Magnesium (mg/100 g) | 354.38 |
| Calcium (mg/100 g) | 819.26 | Zinc (mg/100 g) | 178.49 |
| Iron (mg/100 g) | 59.31 | Vitamin A ($\mu$g/100 g, RE) | 213.11 |
| Phosphorus (mg/100 g) | 187.17 | Vitamin $B_1$(mg/100 g) | 12.29 |
| Vitamin $B_2$ (mg/100 g) | 8.45 | Vitamin $B_6$ (mg/100 g) | 10.50 |
| Vitamin C (mg/100 g) | 4.91 | Vitamin E (mg/100 g, $\alpha$-TE) | 23.61 |
| Vitamin K ($\mu$g/100 g) | 232.12 | Niacin (mg/100 g, NE) | 5.76 |
| Pantothenic acid (mg/100 g) | 5.87 | folic acid ($\mu$g/100 g) | 349.97 |

As seen above, the ginseng berry extract according to the present invention contains more ginseng saponins than the ginseng root extract. The saponin components included in ginseng berry are quite different from those of ginseng root. It was also confirmed that the ginseng berry extract includes 16 vitamins and minerals in large quantity unlike ginseng root. Based on these findings, the following experiments were carried out in order to confirm its effect in blood vessels.

Test Example 2: NO generation in HUVEC (human umbilical vein endothelial cells)

eNOS (endothelial nitric oxide synthase) exists in human endothelial cells. Increased eNOS activity results in NO generation, thereby dilating blood vessels and facilitating blood circulation. Human endothelial cells were cultured and treated with ginseng berry extract (Examples 1 and 2) and red ginseng root extract (Comparative Example 1). NO generation quantity was compared.

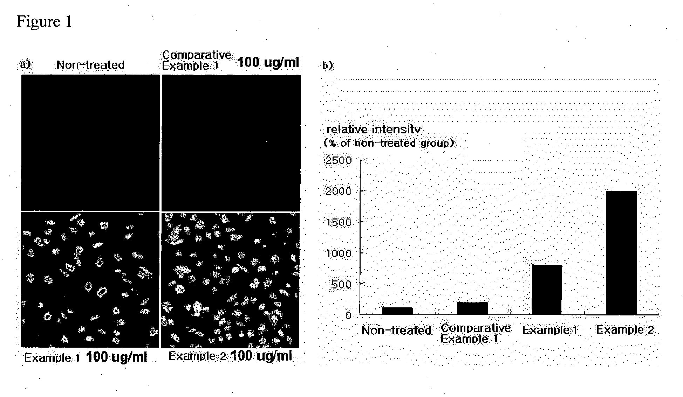

Endothelial cells were adhered on a gelatin-coated 24-well plate, with a density of $2.5 \times 10^4$ cells/well. The cells were cultured for 12 hours using growth medium. The endothelial cells were pre-treated for 12 hours, using ginseng berry extract or red ginseng root extract (Examples 1-2, Comparative Example 1). Then, they, including non-treated group, were treated with 10 μmol/L DAF-FM diacetate (Molecular Probe, OR) at 37 °C for 30 minutes in FBS-free M199 medium. Subsequently, the endothelial cells were washed 3 times with FBS-free M199 medium, put in a parallel plate flow chamber, and stimulated with light isolated from a mercury lamp. Excitation wavelength was 488 nm. Fluorescence of 515 nm is emitted when DAF binds with NO. Figure 1a is a confocal laser microscopic image (Atto Bioscience, USA) of endothelial cells, and Figure 1b compares intensity of fluorescence analyzed using Image-Pro Plus v4.5 software (Media Cybernetics, San Diego, CA, USA) with that of the non-treated group.

As seen in Figure 1, the ginseng berry extracts of Examples 1 and 2 according to the present invention exhibited 1300% to 2000% of NO generation at 100 ug/mL, as compared to the non-treated group. In contrast, the red ginseng root extract of Comparative Example 1 showed about 100% to 200% of NO generation at the same concentration of 100 ug/mL. In particular, the extract of Example 2 exhibited more NO generation. Accordingly, it was confirmed that ginseng berry extract provides significantly better NO generation effect in endothelial cells than red ginseng root extract. The outstanding NO generation ability of the ginseng berry extracts of Examples 1 and 2 results in dilation of blood vessels and facilitates blood circulation.

Comparative Example 3: Viability of endothelial cells

The most basic step of preventing aging of blood vessels and facilitating angiogenesis is activating endothelial cells. Improvement of viability of endothelial cells was compared between the positive control group (VEGF; vascular endothelial growth factor) and ginseng berry extract (Examples 1 and 2) treated groups.

Viability of endothelial cells was measured by crystal violet staining. Endothelial cells were adhered on a 24-well plate, at $5\times10^4$ cells/well, and cultured for 12 hours using growth medium. Then, after treating for 6 hours with M199 medium containing 1% serum, thereby equalizing cell cycle, the cells were treated with the ginseng berry extracts of Examples 1 and 2 at 25, 50 and 100 μg/mL, respectively, or with the positive control substance. After 18 to 24 hours, the medium was removed and 300 μL of crystal violet stain was added to stain living endothelial cells. After leaving at room temperature for about 30 minutes, the cells were washed 2-3 times with phosphate buffered saline (PBS) and lysed with 1% SDS solution. Absorbance at 550 nm was compared between the positive control group and the treatment groups of Examples 1 and 2 to measure viability of cells.

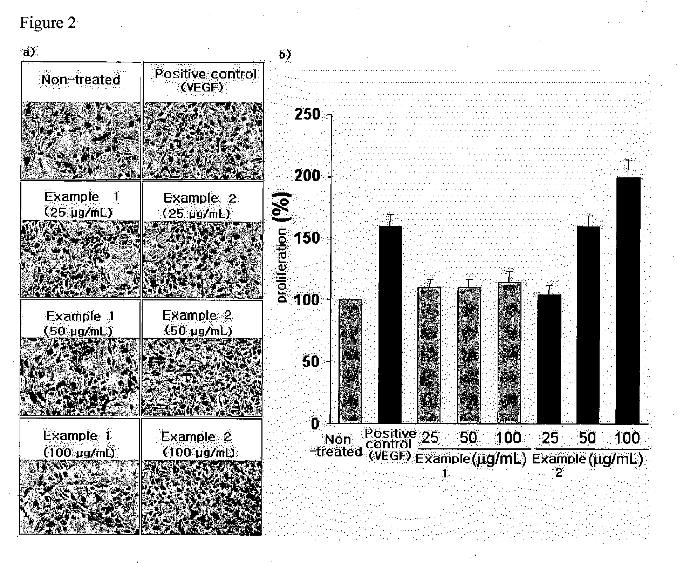

Figure 2a shows surviving endothelial cells, and Figure 2b compares absorbance of stained endothelial cells. As seen in Figure 2a and Figure 2b, the ginseng berry extract (Examples 1 and 2) treatment groups exhibited better viability of endothelial cells and enhanced cell proliferation than the non-treated group. Especially, Example 2 showed viability of endothelial cells even better than the positive control group (VEGF). Therefore, it was confirmed that ginseng berry extract improve viability of endothelial cells and facilitate proliferation thereof, thereby preventing aging of blood vessels and facilitating angiogenesis.

Comparative Example 4: Improvement of endothelial cell mobility (migration of HUVECs)

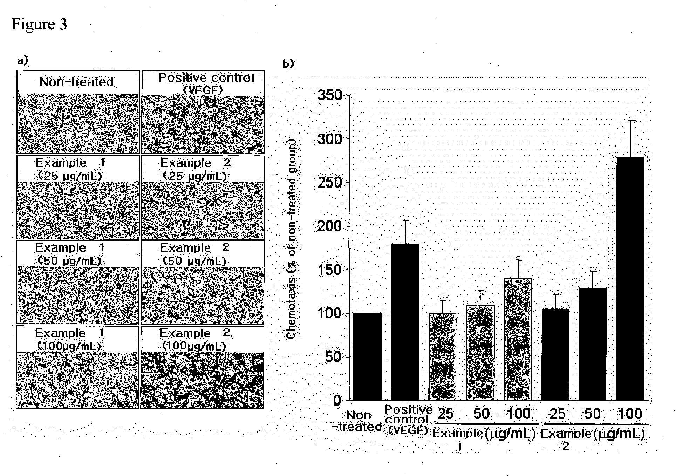

Cell mobility was measured and analyzed as a measure of angiogenesis. Cell mobility analysis was carried out using a Boyden's chamber (transwell). 600 ul of serum-free M199 medium was added to a 24-well plate. After treating the medium with ginseng berry extract (Examples 1 and 2; 25, 50 and 100 μg/mL, respectively) or the positive control substance, gelatin (1 mg/mL) was applied on the lower surface of the transwell and $2\times10^4$ cells were adhered on the upper surface. About 4 hours later, the cells on the upper surface were removed using a swab, and the cells that migrated to the lower surface were counted after staining with hematoxylin and eosin. Figure 3a shows mobility of the stained endothelial cells, and Figure 3b compares mobility of the cells with that of the non-treated group.

As seen in Figure 3, Examples 1 and 2 showed the effect of improving mobility of endothelial cells. Especially, Example 2 showed better effect than the positive control substance VEGF. Accordingly, it can be concluded that ginseng berry extract improves mobility of vascular endothelial cells, which is one of key mechanisms in angiogenesis.

Comparative Example 5 : Facilitation of endothelial cell tube-formation

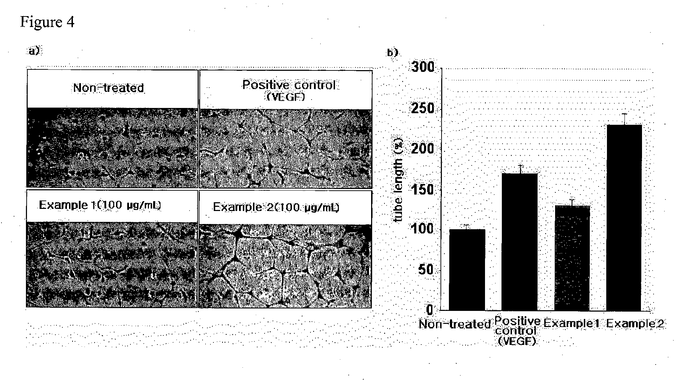

As a measure of angiogenesis control ability, the degree of tube formation by cells was measured. In general, tube formation of cells is tested using Matrigel. 250 ul of Matrigel was coated on a 24-well plate and $2.5\times10^4$ cells were adhered on the Matrigel. With predetermined time intervals, tube formation was observed for the non-treated group, the positive control group (VEGF) and the ginseng berry extract treated groups (Examples 1 and 2). At proper time, cells were fixed and stained and the degree of tube formation was analyzed using a computer program. Figure 4a shows the images of the fixed and stained cells, and Figure 4b compares tube length with that of non-treated group.

As seen in Figure 4, Examples 1 and 2 showed the effect of facilitating tube formation, which is one of key mechanisms in angiogenesis. Especially, Example 2 showed better effect than the positive control substance.

Comparative Example 6: Facilitation of vessel sprouting

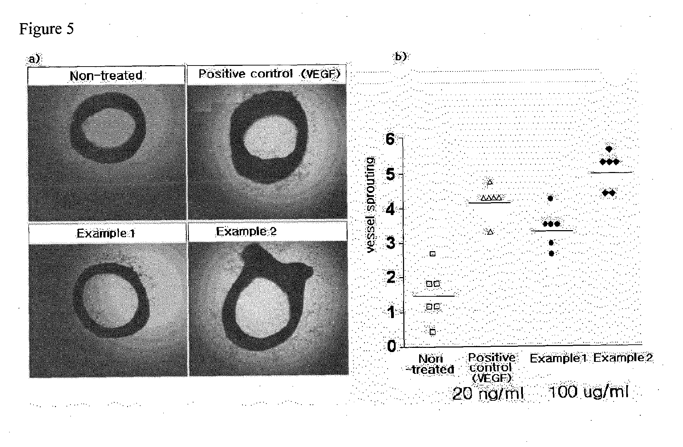

As a measure of angiogenesis control ability, sprouting of vessels was observed. SD rats were used, and the test groups were divided into a non-treated group, a VEGF treated group, and ginseng berry extract (Examples 1 and 2) treated groups. Aortic rings obtained from SD rats were stored in serum-free DMEM medium. The medium in which each of the aortic rings was stored was treated with VEGF or ginseng berry extract (Examples 1 and 2). While culturing at 37 °C for about 7 days, sprouting of blood vessels was observed. Figure 5a is an optical microscopic image showing new capillary vessels extending from the aortic ring, and Figure 5b shows vessel sprouting promoting effect evaluated by measuring length and number of newly formed vessels.

As seen in Figure 5, ginseng berry extract (Examples 1 and 2) showed the effect of facilitating vessel sprouting. Especially, Example 2 showed better effect than the positive control substance. This indicates that ginseng berry extract facilitates angiogenesis.

Comparative Example 7: Facilitation of angiogenesis

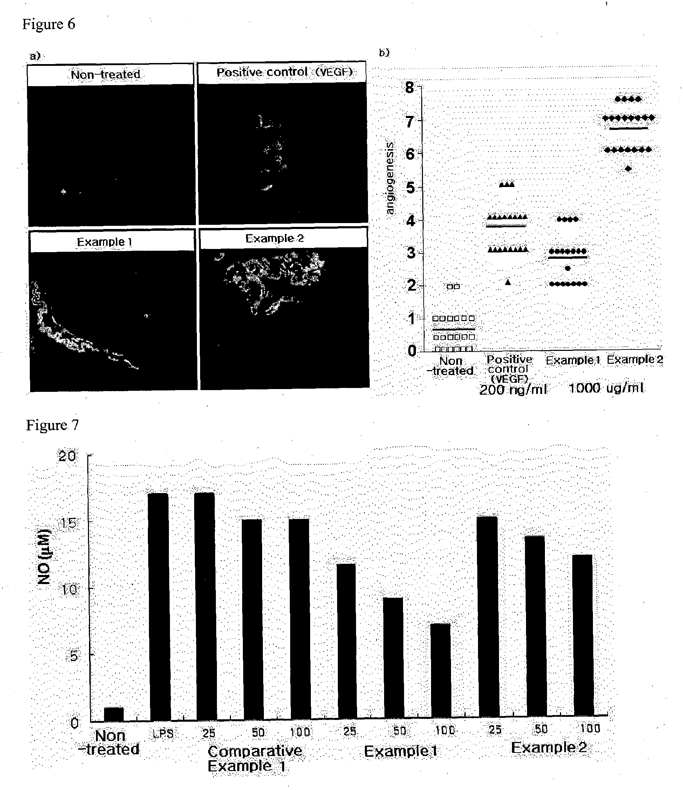

About 6 to 8 weeks-old male BALB/c mouse was anesthetized using 1.5% isoflurane solution and $O_2/N_2O$. At the abdomen of the mouse, a titanium window chamber (diameter = 19 mm, inner diameter = 14 mm, thickness = 0.7 mm) was implanted. Each of 20 ng of VEGF, as control, and ginseng berry extract (Examples 1 and 2) was mixed with Matrigel and placed in the tissue inside the window. The cover slip was covered and fixed with a snap ring. Angiogenesis was observed in real time through intravital fluorescence microscopy. 4 days later, 50 $\mu$L of dextran (MW 250,000, Sigma Chemical, St. Louis, Missouri) labeled with fluorescein isothiocyanate (FITC) was injected into the tail vein at a concentration of 25 mg/mL, in order to confirm the degree of angiogenesis. Figure 6a is a real-time, intravital fluorescence microscopic image (Zeiss Axiovert 200M microscopy, Oberkocchen, Germany) taken with an electron-multiplying CCD camera (Photon Max 512; Princeton Instruments, Trenton, NJ) using blue light (excitation at 440-475 nm, emission at 530-550 nm), and Figure 6b shows blood vessel change analyzed using the computer program MetaMorph (Universal Imaging Corp., Downingtown, PA) (0: fewest changes, 5: most changes).

As seen in Figure 6, ginseng berry extract (Examples 1 and 2) showed angiogenic effect in animal. Especially, Example 2 showed better angiogenic effect than the positive control substance. Accordingly, it can be confirmed that ginseng berry extract is effective in facilitating angiogenesis and, thus, is effective in treating ischemic cardiovascular disease, improving local blood circulation and treating chronic vascular inflammation.

Comparative Example 8: Control of vascular inflammation

1. Effect of inhibiting LPS-induced NO generation in macrophages (in vitro)

Effect of inhibiting LPS-induced NO generation in macrophages was tested in order to confirm the effect of inhibiting vascular inflammation of ginseng berry extract.

Macrophages (Raw 264.7 cells) were cultured in a medium containing 10% serum under the condition of 5% $CO_2$. After culturing on a 96-well plate incubator to a concentration of $2 \times 10^5$ cells/well and stimulating with LPS (1 ug/mL), the cells were treated with ginseng berry extract (Examples 1 and 2) or red ginseng root extract (Comparative Example 1), respectively. After keeping at 37 °C for 1 hour, the degree of NO generation was measured to compare the effect of inhibiting LPS-induced NO generation. NO generation was measured using the Griess reaction [Minghetti, L. et al., 1991, Glia 19. 152-160]. The result is shown in Figure 7.

As seen in Figure 7, the ginseng berry extract according to the present invention (Examples 1 and 2) effectively inhibited generation of NO, as cytokine, and the effect was remarkably superior to that of the red ginseng root extract (Comparative Example 1). Especially, Example 1 showed better effect. Accordingly, it can be confirmed that the ginseng berry extracts according to the present invention (Examples 1 and 2) are effective in alleviating vascular inflammation and treating vascular inflammation-related ischemic disease such as angina pectoris.

2. Effect of controlling vascular inflammation (in vivo)

Based on the experimental result in the cell level, the effect of controlling vascular inflammation was tested in animal model. Mice were grouped into a non-treated group, an LPS-induced group, and ginseng berry extract (Examples 1 and 2) treated groups, with 5 mice per each group.

100 mg of each of the ginseng berry extracts prepared in Examples 1 and 2 per 1 kg body weight of mouse (1.5 mg/15 g body weight of mouse) was diluted in 1 mL of saline solution (0.9% NaCl). This solution was intra-abdominally injected three times from day 1 through day 3. 2 hours after the injection on day 3, LPS diluted in 1 mL of saline solution at a concentration of about 4 mg per kg body weight of mouse was injected intra-abdominally to induce inflammation.

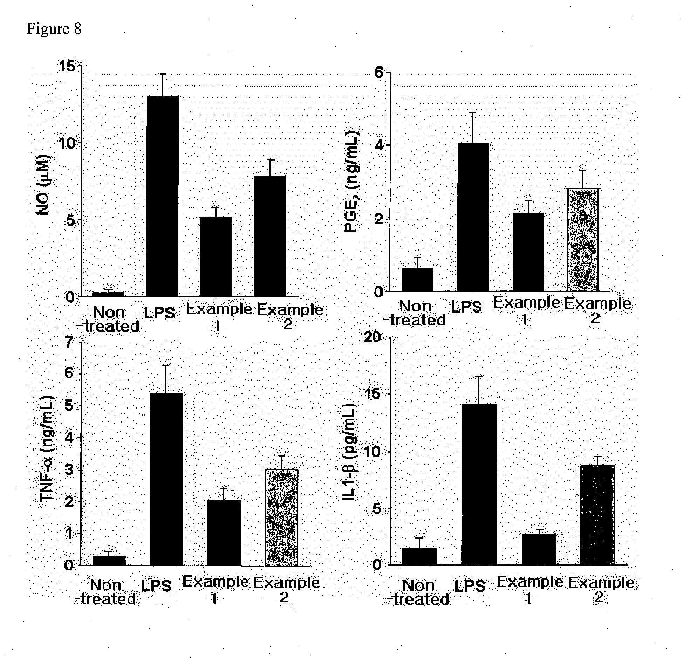

After approximately 12 hours, the abdomen of the mouse was cut open, and blood was taken from the artery of the heart and stored at -40 °C. The blood was kept at room temperature for 30 minutes and, after centrifuge at 4 °C, 3,000 rpm for 10 minutes, the supernatant was kept at -20 °C. NO generation was determined from the blood, and inflammatory factors PGE2, TNF-α and IL1-β were identified by Western blotting. The result is shown in Figure 8.

As seen in Figure 8, ginseng berry extract (Examples 1 and 2) showed the effect of inhibiting LPS-induced inflammation. Especially, Example 1 showed better effect. This indicates that ginseng berry extract is effective in inhibiting vascular inflammation and, thereby, controlling vascular inflammation in ischemic disease.

Comparative Example 9: Inhibition of skin aging and improvement of wrinkles

1) Antioxidative effect

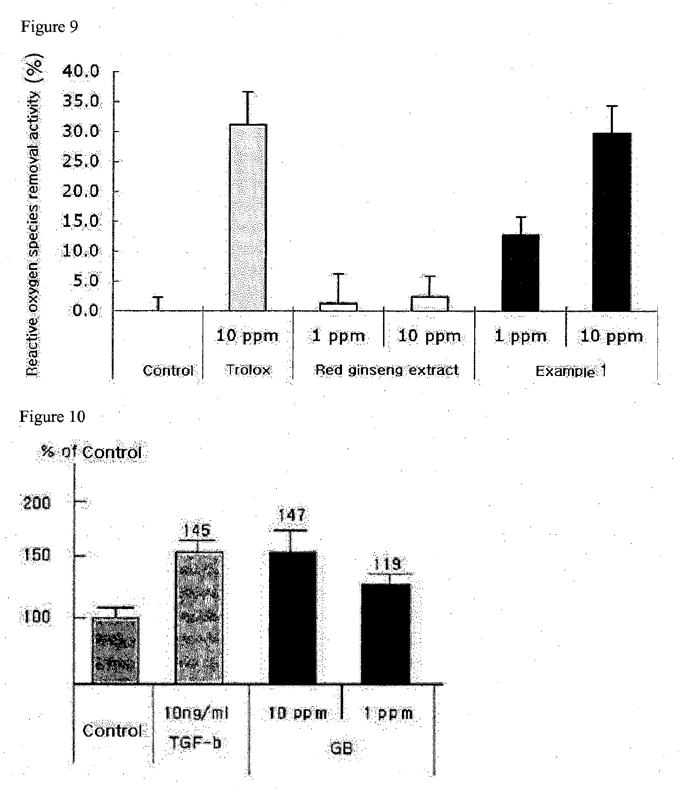

Antioxidative effect was investigated by comparing the ability of removing reactive oxygen species (ROS) generated in cells by ultraviolet (UV) radiation. Trolox, which is commonly used to compare antioxidative effect, was used as positive control substance. And, red ginseng extract was used for the purpose of comparison, and non-treated group was used as control group (Figure 9).

As seen in Figure 9, the ginseng berry extract of according to the present invention (Example 1) significantly scavenged ROS generated by UV radiation in a human HaCaT keratinocytes monolayer culture system, as compared to the control group. The effect was comparable to that of trolox, which is used as antioxidative activity index. In contrast, the red ginseng extract did not show a significant scavenging effect. Accordingly, it can be confirmed that the ginseng berry extract of the present invention (Example 1) significantly scavenges ROS, which is a cause of skin aging and, thus, is effective in preventing wrinkles, decrease of skin elasticity, pigmentation, and the like.

2) Type I procollagen assay

Human fibroblasts were cultured on a 12-well plate incubator to a concentration of $10^5$ cells/well. Then, the medium was replaced by one including 1 ppm or 10 ppm of the ginseng berry extract of Example 1. On the 3rd day of culturing, the cells were harvested and the quantity of produced type I procollagen was analyzed using ELISA. The result was calculated as a relative value based on the control group, which was not treated with a test substance. TGF-b (transforming growth factor-b) was used as positive control substance.

In normal human fibroblast monolayer culture system, the ginseng berry extract of Example 1 showed a distinct effect of facilitating type I procollagen generation as compared to the control group. That is, it can be confirmed that the ginseng berry extract of Example 1 can inhibit the reduction of collagen generation due to aging of human skin and can improve wrinkles (Figure 10).

3) Inhibition of MMP-1 expression

Human fibroblasts were cultured on a 12-well plate incubator to a concentration of $10^5$ cells/well. Then, after irradiating with UVB at 40 mJ/cm$^2$, the medium was replaced by one including 1 ppm or 10 ppm of the ginseng berry extract of Example 1. On the 2nd day of culturing, the cells were harvested and the quantity of produced MMP-1 (matrix metalloproteinase I) was analyzed using ELISA. The result was calculated as a relative value based on the control group, which was irradiated with UV without treating with test substance. TGF-b (transforming growth factor-b) was used as positive control substance.

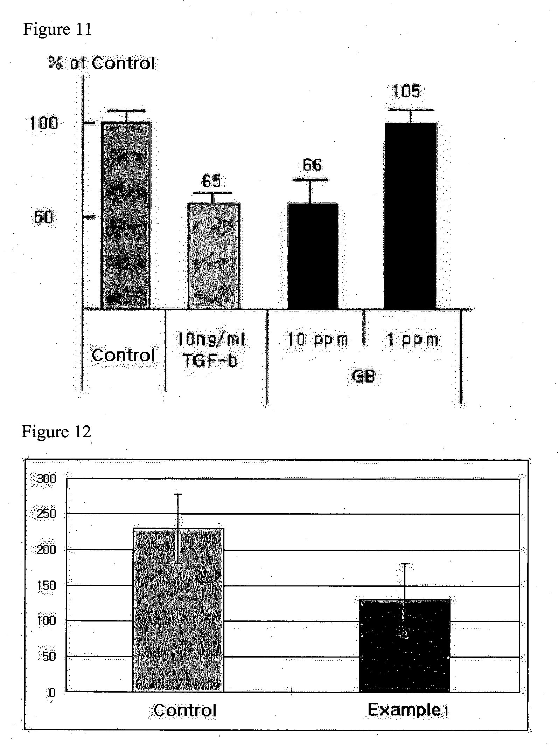

In normal human fibroblast monolayer culture system, the ginseng berry extract of Example 1 significantly inhibited the expression of MMP-1 induced by UVB 40 mJ/cm$^2$ radiation. That is, it can be confirmed that the ginseng berry extract of Example 1 can inhibit the biosynthesis of MMP-1, which is the enzyme involved in the breakdown of skin tissue, induced by internal or external aging factors and, thus, is effective in preventing skin aging and improving wrinkles (Figure 11).4) Inhibition of biosynthesis of cyclooxygenase-2 (COX-2) induced by UV

Human fibroblasts were cultured on a 12-well plate incubator to a concentration of $10^5$ cells/well. Then, after irradiating with UVA at 15 J/cm$^2$, the medium was replaced by one including each 0.1 ppm, 1 ppm or 10 ppm of the ginseng berry extract of Example 1 or the red ginseng root extract of Comparative Example 1. On the 2nd day of culturing, the cells were harvested and the quantity of produced COX-2 was analyzed using Western blotting. The result was calculated as a relative value based on the control group, which was irradiated with UV without treating with test substance, using a densitometer. The result is given in the following Table 3.

Table 3

| Test substance (ppm) | | COX-2 biosynthesis (%) |
|---|---|---|
| Example 1 | 10 | 11 |
| | 1 | 38 |
| | 0.1 | 63 |
| Comparative Example 1 | 10 | 72 |
| | 1 | 91 |
| | 0.1 | 99 |
| Control | | 100 |

As seen from Table 3, the ginseng berry extract of Example 1 decreased biosynthesis of COX-2 induced by UV in a concentration-dependent manner. As a result, it can prevent the breakdown of skin tissue by prostaglandin E2 (PGE2) derived from COX-2.

5) Inhibition of biosynthesis of tumor necrosis factor-$\alpha$(TNF-$\alpha$) induced by UV

Human keratinocytes were cultured on a 12-well plate incubator to a concentration of $10^5$ cells/well. Then, after irradiating with UVB at 30m J/cm$^2$, the medium was replaced by one including each 0.1 ppm, 1 ppm or 10 ppm of the ginseng berry extract of Example 1 or the red ginseng extract Comparative Example 1. After 6 to 24 hours of culturing, the cells were harvested and the quantity of TNF-$\alpha$ was analyzed using ELISA (Pharmingen 555212). The result was calculated as a relative value based on the control group, which was irradiated with UV without treating with test substance. The result is given in the following Table 4.

Table 4

| Test substance (ppm) | | TNF-$\alpha$ synthesis (%) |
|---|---|---|
| Example 1 | 10 | 16 |
| | 1 | 38 |
| | 0.1 | 72 |
| Comparative Example 1 | 10 | 74 |
| | 1 | 85 |
| | 0.1 | 92 |
| Control | | 100 |

As seen from Table 4, the ginseng berry extract of Example 1 according to the present invention decreased biosynthesis of TNF-$\alpha$ induced by UV in a concentration-dependent manner. Therefore, it was confirmed that ginseng berry extract can prevent skin aging, which may be caused by the biosynthesis of TNF-$\alpha$.

6) Inhibition of skin aging

Hairless mice were selected as animal model for evaluating whether the composition of the present invention has skin aging prevention effect. 6 to 7 weeks-old female mice were grouped as a normal feed group (control) and a normal feed + ginseng berry extract (Example 1) group, with 10 mice per each group. After 12 weeks of oral administration, the mice were irradiated with UV.

Skin wrinkles before and after UV radiation were compared in the same region using Visiometer system (C+K). Change of skin wrinkles was calculated by the following Math Figure 1. The result is shown in Figure 12.

Math Figure 1

$$\text{Change of wrinkles } (\Delta\%) = \frac{Tdi - Tdo}{Tdo} \times 100$$

where Tdi is the measurement value at Day 90, and Tdo is the measurement value at Day 0.

As seen in Figure 12, the test group to which normal feed and the ginseng berry extract of Example 1 were given showed 130±51% (mean ± standard deviation) of skin wrinkle increase, whereas the control group to which normal feed was given showed 230±49% of skin wrinkle increase. Therefore, the test group showed better skin aging inhibition effect (Figure 12).

7) Improvement of wrinkles

Hairless mice were selected as animal model for evaluating whether the composition of the present invention provides the effect improving existing wrinkles. 6 to 7 weeks-old female mice were grouped as a normal feed group (control) and a normal feed + ginseng berry extract (Example 1) group, with 10 mice per each group. After irradiation with UV to induce skin wrinkles, the mice were orally administered with the feed and the extract for 12 weeks.

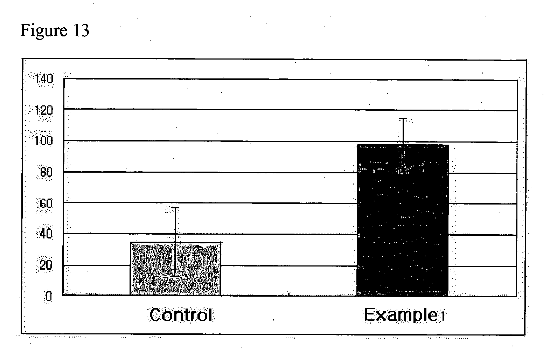

Skin wrinkles before and after oral administration were compared in the same region using Visiometer system (C+K). Change of skin wrinkles was calculated by the following Math Figure 2. The result is shown in Figure 13.

Math Figure 2

$$\text{Change of wrinkles } (\Delta\%) = \frac{Tdi - Tdo}{Tdo} \times 100$$

where Tdi is the measurement value at Day 90, and Tdo is the measurement value at Day 0.

As seen in Figure 13, the test group to which normal feed and the ginseng berry extract of Example 1 were given showed 98±17% (mean ± standard deviation) of skin wrinkle decrease, whereas the control group to which normal feed was given showed 35±22% of skin wrinkle decrease. Therefore, the test group showed better skin wrinkle improving effect.

Comparative Example 10: Skin whitening effect

1) Inhibition of melanin generation in mouse melanocytes

Inhibition of melanin generation in mouse melanocytes was evaluated in order to confirm the effect of the ginseng berry extract of Example 1 of inhibiting melanin generation.

First, melanocytes (Mel-Ab cells) derived from C57BL/6 mouse [Dooley, T.P. et al, Skin Pharmacol., 7, pp. 188-200] were cultured in DMEM (Dulbecco's modified Eagle's medium) including 10% FBS, 100 nM 2-O-tetradecanoylphorbol-13-acetate and 1 nM cholera toxin under the condition of 37 °C, 5% $CO_2$. The cultured Mel-Ab cells were separated using 0.25% trypsin-EDTA, and cultured on a 24-well plate to a concentration of $10^5$ cells/well. Starting from day 2, 10 ppm of the ginseng berry extract of Example 1 was added for 3 consecutive days. Red ginseng extract was used for comparison, and hydroquinone was used as positive control substance. Then, after removing the medium and washing with PBS, the cells were dissolved with 1 N sodium hydroxide. Absorbance was measured at 400 nm, and melanin generation inhibition ratio was calculated by the following Math Figure 3. The result is given in the following Table 5 (Dooley's method).

Math Figure 3

$$\text{Melanin generation inhibition ratio (\%)} = 100 - \left( \frac{\text{Absorbance of test substance}}{\text{Absorbance of control}} \times 100 \right)$$

Table 5

| Test substance | Melanin generation inhibition ratio (%) |
|---|---|
| Example 1 (100 ppm) | 69 |
| Example 1 (10 ppm) | 37 |
| Example 1 (1 ppm) | 10 |
| Control (red ginseng extract, 10 ppm) | 5 |
| Positive control (hydroquinone, 10 ppm) | 39 |

As seen from Table 5, the ginseng berry extract of Example 1 showed melanin generation inhibition ratio better than red ginseng extract (control group) and comparable to hydroquinone (positive control).

2) Skin whitening effect through oral administration

Brown guinea pig was selected as animal model in order to confirm whether the composition of the present invention provides skin whitening effect as health functional food. Animals in all test groups (10 animals per each group) were irradiated with UV and minimum erythemal dose was measured. All the animals were irradiated with UV at the minimum erythemal dose for 3 days, once a day. Test animals were divided into a normal feed group, a normal feed + red ginseng extract group, and a normal feed + ginseng berry extract (Example 1) group. The animals were freely allowed to feed for 5 weeks. Pigmentation was evaluated by measuring L value (brightness) with a colorimeter, and calculating difference between the L value on each day and that before UV radiation. The result is given in the following Table 6.

Table 61

| UV radiation | Normal feed | Normal feed + red ginseng extract | Normal feed + Example 1 |
|---|---|---|---|
| 1 week later | 7.03±0.28 | 6.90±0.48 | 6.92±0.30 |
| 2 weeks later | 6.61±0.31 | 6.50±0.11 | 6.04±0.18 |
| 3 weeks later | 6.42±0.26 | 5.91±0.26 | 4.56±0.25 |
| 4 weeks later | 6.16±0.45 | 5.66±0.11 | 3.44±0.16 |
| 5 weeks later | 6.01±0.49 | 5.42±0.23 | 2.88±0.15 |

As seen from Table 6, the test group to which the ginseng berry extract of Example 1 according to the present invention was administered exhibited fast reduction of change of L value. This means that, in the group to which the ginseng berry extract of Example 1 according to the present invention was administered, the darkened skin returns to the original skin color faster that the group to which the red ginseng extract was given. It was confirmed that the ginseng berry extract of Example 1 according to the present invention provides superior skin whitening effect to red ginseng extract.

Comparative Example 11: Skin moisturizing effect

1) Facilitation of hyaluronic acid producing ability

Hyaluronic acid is a link protein which keeps moisture in the intercellular space and is directly related with skin moisturizing effect.

Human epidermal cells were cultured and the medium was replaced by either a medium for culturing of epidermal cells (control group) or one including the ginseng berry extract of Example 1. Under the same conditions, the cells were cultured for 48 hours. Then, the quantity of hyaluronic acid in the two groups was compared using a hyaluronic acid measurement kit. The result was given as relative value based on the control group.

Table 7

| Test substance | Concentration | Facilitation of |
|---|---|---|
| | | hyaluronic acid production (%) |
| Example 1 | 100 ppm | 189 |
| | 10 ppm | 134 |
| | 1 ppm | 112 |
| Control | - | 100 |

As seen from Table 7, the ginseng berry extract of Example 1 facilitated the production of hyaluronic acid in the epidermal cells. Therefore, it was confirmed that the ginseng berry extract of Example 1 according to the present invention provides skin moisturizing effect.

2) Skin moisturizing effect through oral administration

Hairless mice were selected as animal model for evaluating whether the composition of the present invention provides skin moisturizing effect. 6 to 7 weeks-old female mice were grouped into a normal feed group, a normal feed + red ginseng extract group, and a normal feed + ginseng berry extract (Example 1) group, with 10 mice per each group. After 12 weeks of oral administration, the quantity of hyaluronic acid in the epidermis and the dermis was compared using a hyaluronic acid measurement kit. The result is given in the following Table 8.

Table 8

| | Normal feed | Normal feed + red ginseng extract | Normal feed + Example 1 |
|---|---|---|---|
| Hyaluronic acid content ($\mu$g/g) | $502.80 \pm 16.24$ | $539.33 \pm 36.11$ | $617.89 \pm 20.21$ |

As seen from Table 8, the test group to which the ginseng berry extract of Example 1 was administered exhibited increased hyaluronic acid content in the epidermis and the dermis. The skin moisturizing effect was superior than in the group to which a normal feed was administered and the group to which normal feed and red ginseng extract were administered.

Test Example 12: Increase of NO generation in endothelial cells

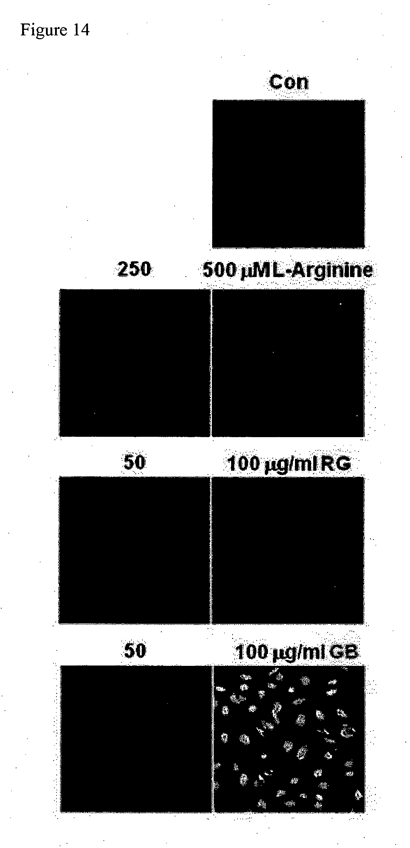

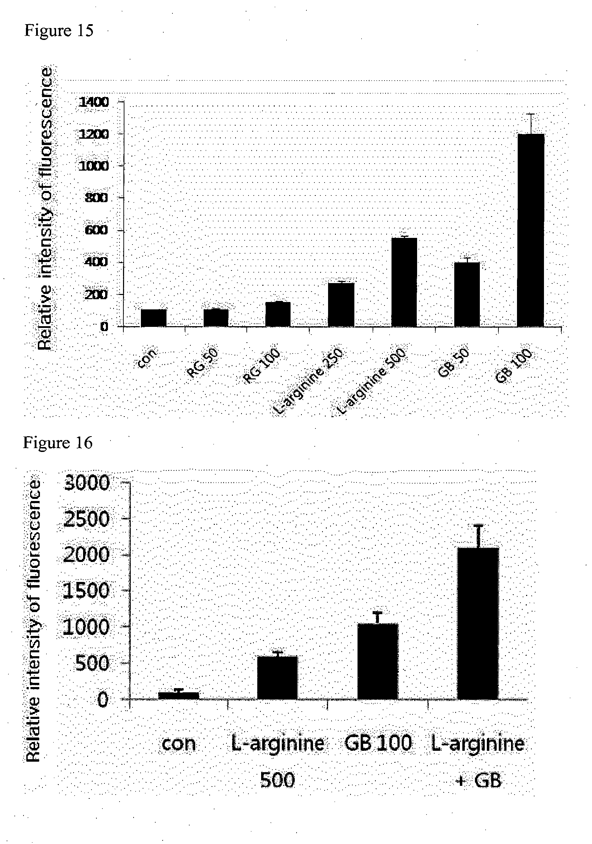

Increase of generation of NO, which is known as an important signal transduction substance related with penial erection, in endothelial cells was observed after treating with ginseng berry extract. Experiment was carried out in a manner similar to Test Example 2, but 1-arginine was treated together. Figure 14 is a confocal laser microscopic image (Atto Bioscience, USA), and Figure 15 is a graph of relative fluorescence intensity analyzed with Image-Pro Plus v4.5 software (Media Cybernetics, San Diego, CA, USA). In Figure 14 and Figure 15, "con" stands for control, "RG" for the group treated with red ginseng extract of Comparative Example 1, "GB" for the group treated with the ginseng berry extract of Example 1, "RG 50" for the group treated with red ginseng extract 50 $\mu$g/mL, "RG 100" for the group treated with red ginseng extract 100 $\mu$g /mL, "L-arginine 250" for the group treated with 1- arginine 250 $\mu$M, "L-arginine 500" for the group treated with 1-arginine 500 $\mu$M, "GB 50" for the group treated with ginseng berry extract 50 $\mu$g/mL, and "GB 100" for the group treated with ginseng berry extract 100 $\mu$g/mL.

As seen from Figure 14 and Figure 15, when treated with ginseng berry extract, the endothelial cells (HUVEC) exhibited significantly increased NO generation in monolayer culture system, as compared to the control group. The effect was concentration-dependent. At a concentration of 100 $\mu$g/mL, the red ginseng group exhibited about 1.5 times of increased NO generation to the control group. 1-Arginine, which is the substrate of nitric oxide synthase, increased NO generation by about 2.7 times and 5.5 times, at 250 $\mu$M and 500 $\mu$M, respectively. In contrast, ginseng berry extract increased generation of NO, which is a blood vessel dilation signal transduction substance, by about 4 times and 12 times, as compared to the control group, at 50 $\mu$g/mL and 100 $\mu$g/mL, respectively. That is, the ginseng berry extract exhibited the best effect. The ginseng berry extract was about 8 times more efficient than the red ginseng extract, at a concentration of 100 $\mu$g/mL.

Therefore, it can be confirmed that, intake of ginseng berry extract results in increased NO generation in endothelial cells and, thus, can improve penial erection through dilation of blood vessels in the corpus cavernosum.

Test Example 13: Synergic effect of facilitating NO generation upon combined use of ginseng berry extract and 1-arginine

It was observed whether a combination of 1-arginine, which is known as the substrate of nitric oxide synthase, and ginseng berry extract, which facilitates NO generation, provides a synergic effect of facilitating NO generation.

Endothelial cells were isolated from the umbilical cord and cultured. NO generation in the cultured endothelial cells was measured. Specific experimental procedure was the same as in Test Example 12. The result is shown in Figure 16.

In Figure 16, "con" stands for control, "L-arginine 500" for 1-arginine 500 $\mu$M, "GB 100" for ginseng berry extract 100 $\mu$g/mL, and "L-arginine + GB" for 1-arginine 500 $\mu$M + ginseng berry extract 100 $\mu$g/mL.

As seen in Figure 16, NO generation increased much more when the endothelial cells were treated with 100 $\mu$g/mL ginseng berry extract and 500 $\mu$M 1-arginine at the same time, than when they were treated with either of the two. That is, a synergic effect was confirmed.

From this result, it can be confirmed that a combined use of 1-arginine, which is the substrate of nitric oxide synthase, and ginseng berry extract greatly increases NO generation and, thus, is effective in improving male sexual function through relaxation of the corpus cavernosum and improved and maintained penial erection.

Hereunder are described some formulation examples of the composition of the present invention. However, they are described for illustrative purposes and are not intended to limit the scope of the present invention.

Formulation Example 1: Injection

50 mg of the ginseng berry extract of Example 1, adequate amount of sterilized water for injection, and adequate amount of pH adjuster were mixed and filled in an ampule (2 mL) according to a common injection preparation method.

Formulation Example 2: Liquid

100 mg of the ginseng berry extract of Example 2, 10 g of isomerized glucose, and 5 g of mannitol were dissolved in adequate amount of purified water. After adding adequate amount of lemon flavor and mixing the ingredients, purified water was added to make 100 mL. The resultant liquid was filled in a brown bottle and sterilized.

Formulation Example 3: Soft capsule

50 mg of the ginseng berry extract of Example 1, 80-140 mg of 1-carnitine, 180 mg of soybean oil, 2 mg of palm oil, 8 mg of hardened vegetable oil, 4 mg of yellow beeswax, and 6 mg of lecithin were mixed and filled in a capsule according to a common method, with 400 mg per each capsule.

Formulation Example 4: Tablet

50 mg of the ginseng berry extract of Example 2, 200 mg of galactooligosaccharide, 60 mg of lactose, and 140 mg of maltose were mixed and granulated using a fluidized bed drier. Then, after adding 6 mg of sugar ester, the mixture was prepared into tablet using a tablet making machine.

Formulation Example 5: Granule

50 mg of the ginseng berry extract of Example 2, 250 mg of glucose anhydrocrystalline, and 550 mg of starch were mixed and granulated using a fluidized bed granulator.

Formulation Example 6: Drink

50 mg of the ginseng berry extract of Example 1, 10 g of glucose, 0.6 g of citric acid, and 25 g of oligosaccharide syrup were mixed. After adding 300 mL of purified water, 200 mL of the mixture was filled in a bottle. Then, sterilization was carried out at 130 °C for 4-5 seconds.

Formulation Example 7: Ginseng berry 100% extract

During the ginseng berry extract preparation process in Example 1, the extract was concentrated so that the solid content is 60% or higher. After aging at low temperature, 100% extract liquid product was prepared.

Formulation Example 8: Pill

0.9 g of the ginseng berry extract of Example 1, 0.3 g of sugar, 1.91 g of starch, and 0.56 g of glycerin were mixed and prepared into pills using a pill making machine.

**Claims**

1. A composition comprising berry extract of *Panax ginseng* C.A. Meyer as active ingredient for therapeutic use in the improvement of male sexual function.

2. The composition for use according to claim 1, wherein the composition further comprises L-arginine.

3. The composition for use according to claim 1,
   wherein the composition comprises 0.01-100 weight% of the ginseng berry extract based on the total weight of the composition.

4. The composition for use according to claim 2,
   wherein the composition comprises 0.01-99.9 weight% of the L-arginine based on the total weight of the composition.

5. The composition for use according to any one of claims 1 to 4, wherein the composition increases generation of nitric oxide (NO) in vascular endothelial cells.

6. The composition for use according to any one of claims 1 to 5, wherein the composition improves penile erection.

7. The composition for use according to any one of claims 1 to 4,
   wherein the composition is in the preparation form of tablet, pill, pellet, capsule, granule, powder, ointment, drink or injection.

**Patentansprüche**

1. Eine Zusammensetzung , die als aktiven Bestandteil einen Beerenextrakt aus *Panax ginseng* C.A. Meyer zur therapeutischen Verwendung für die Verbesserung der männlichen Sexualfunktion umfasst.

2. Zusammensetzung zur Verwendung nach Anspruch 1, wobei die Zusammensetzung ferner L-Arginin umfasst.

3. Zusammensetzung zur Verwendung nach Anspruch 1, wobei die Zusammensetzung bezüglich des Gesamtgewichts der Zusammensetzung 0,01 - 100 Gew.-% des Ginsengbeerenextrakts umfasst.

4. Zusammensetzung zur Verwendung nach Anspruch 2, wobei die Zusammensetzung bezüglich des Gesamtgewichts der Zusammensetzung 0,01- 99,9 Gew.-% des L-Arginin umfasst.

5. Zusammensetzung nach einem beliebigen der Ansprüche 1 bis 4, wobei die Zusammensetzung die Produktion von Stickstoffmonoxid (NO) in vaskulären Endothelzellen erhöht.

6. Zusammensetzung nach einem beliebigen der Ansprüche 1 bis 5, wobei die Zusammensetzung die Erektion des Penis verbessert.

7. Zusammensetzung nach einem beliebigen der Ansprüche 1 bis 4, wobei die Zusammensetzung in der Verabreichungsform von Tabletten, Pillen, Pellets, Kapseln, Granulat, Pulver, Salbe, Getränk oder als Injektion vorliegt.

**Revendications**

1.  Une composition comprenant un extrait de baies de *Panax ginseng* C.A. Meyer comme ingrédient actif pour une utilisation thérapeutique dans l'amélioration de la fonction sexuelle masculine.

2.  La composition pour une utilisation selon la revendication 1, dans laquelle la composition comprend en outre de la L-arginine.

3.  La composition pour une utilisation selon la revendication 1, dans laquelle la composition comprend de 0,01-100 % en poids d'un extrait de baies de ginseng par rapport au poids total de la composition.

4.  La composition pour une utilisation selon la revendication 2, dans laquelle la composition comprend de 0,01-99,9 % en poids de L-arginine par rapport au poids total de la composition.

5.  La composition pour une utilisation selon l'une quelconque des revendications 1 à 4, dans laquelle la composition augmente la génération d'oxyde nitrique (NO) dans les cellules endothéliales vasculaires.

6.  La composition pour une utilisation selon l'une quelconque des revendications 1 à 5, dans laquelle la composition améliore l'érection du pénis.

7.  La composition pour une utilisation selon l'une quelconque des revendications 1 à 4, dans laquelle la composition est sous la forme de préparation d'un comprimé, d'une pilule, d'une pastille, d'une capsule, d'une granule, d'une poudre, d'une pommade, d'une boisson ou d'une injection.

Figure 1

Figure 2

Figure 3

Figure 4

Figure 5

Figure 6

a)

b)

Figure 7

Figure 8

Figure 9

Figure 10

Figure 11

Figure 12

Figure 13

Figure 14

Figure 15

Figure 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- The Recent Korean Ginseng: Constituents and Effects. Korea Ginseng and Tobacco Research Institute, 1996, 56-112
- **ATTELE AS et al.** *Biochem. Pharmacol.,* 1999, vol. 58, 1685-1693
- **DEY L. et al.** *Phytomedicine,* 2003, vol. 10, 600-605
- The Recent Korean Ginseng: Cultivation. Korea Ginseng and Tobacco Research Institute, 1996, 130-131
- **FORETE, P. et al.** Basal nitric acid synthesis in essential hypertension. *Lancet,* 1997, vol. 349, 837-842
- **CROSSMAN, DC.** More problems with endothelium. *Q J Med.,* vol. 90, 157-160
- **FOLKMAN, J. et al.** *Angiogenesis. The Journal of Biological Chemistry,* 1992, vol. 267 (16), 10931-10934
- **JEONG, JIN-OK et al.** Therapeutic angiogenesis. *Journal of Korean Society for Vascular Surgery,* 2000, vol. 16 (2), 265-269
- **SIMONSEN et al.** Nitric oxide is involved in the inhibitory neurotransmission and endothelium-dependent relaxations of human small penial arteries. *Clin. Sci.,* vol. 92 (3), 265-75
- **CHUANG et al.** cGMP mediates corpus cavernosum smooth muscle relaxation with altered cross-bridge function. *Life Sci.,* 1998, vol. 63 (3), 185-94
- **DOOLEY, T.P. et al.** *Skin Pharmacol.,* vol. 7, 188-200